# EUROPEAN PATENT APPLICATION

(11) **EP 1 038 878 A1**
(43) Date of publication of application: **27.09.2000**
(21) Application number: 00105365.1
(22) Date of filing: 17.03.2000
(51) Int. Cl.: C07H 3/04

(54) **Method of releasing saccharide from glycoside**

(30) Priority: 19.03.1999 JP 7557599
(71) Applicant: AMANO PHARMACEUTICAL CO., LTD., Nishikasugai-gun, Aichi (JP)
(72) Inventor: Yamamoto, Shigeru, Amano Pharm.Co.,Ltd., Kenkyusho, Nishiharu-cho, Nishikasugai-gun, Aichi (JP)
(74) Representative: HOFFMANN - EITLE

(57) **Abstract**

An enzyme composition which acts on a disaccharide glycoside, e.g., β-primeveroside or its analog, to thereby form a physiologically active component, and a method of releasing one or more monosaccharide unit from a glycoside (for example, β-primeveroside) by treating the glycoside with an enzyme composition. The enzyme composition comprises at least one of β-xylosidase and β-glucosidase.

## Description

### FIELD OF THE INVENTION

This invention relates to enzyme compositions containing as the active ingredient enzymes which can be obtained from microorganisms or plants and are capable of acting on β-primeveroside or its analogues serving as precursors at scent components, pigment components or physiologically active components to thereby form various physiologically active components. More particularly, it relates to use of enzyme compositions containing β-xylosidase and/or β-glucosidase capable of cleaving one or more monosaccharide unit from a disaccharide glycoside (e.g., β-primeveroside or its analogs) to thereby release physiologically active components.

### BACKGROUND OF THE INVENTION

Vegetable scent components (for example, geraniol, linalol, benzyl alcohol, 2-phenyl ethanol) and alcoholic scent components (for example, C13-norterpenoid alcohol) play important roles in generating the scents of flowers, tea, fruits and wine.

Monosaccharide glycosides such as β-D-glucopyranoside have been isolated and identified as precursors for benzyl alcohol and (Z)-3-hexenol among these scent components.

Recently, there has been confirmed that a disaccharide glycoside, β-primeveroside (6-O-β-D-xylopyranosyl-β-D-glucopyranoside) or its analogs, exists as precursors for alcoholic scent components such as geraniol and linalol which is considered to play important roles in the scents of, for example, flowers. Also, the presence of the disaccharide glycoside, β-primeveroside and its analogs, has been clarified as precursors for other alcoholic scent components described above.

In addition, it has been revealed that the β-primeveroside and its analogs also occur as the precursors for some physiologically active components other than scent components (for example, pigment and pharmacological components). For example, it is known that macrozamin contained in, for example, cycad is cleaved with β-primeverosidase in a disaccharide unit to give a physiologically active component methylazoxymethanol.

As described above, only few enzymes capable of acting on a disaccharide glycoside, β-primeveroside and its analogs, which are physiologically active component precursors and releasing physiologically active components have been confirmed and purified from tea leaves. Therefore, it has been urgently required to develop an enzyme capable of acting on a disaccharide glycoside, β-primeveroside or its analog, and thus releasing physiologically active components without being restricted in the source to, for example, tea leaves.

### SUMMARY OF THE INVENTION

To solve these problems, the present inventors have conducted intensive studies and consequently found out that an enzyme composition containing conventionally known enzymes is unexpectedly capable of acting on the disaccharide glycoside, β-primeveroside or its analog, and thus releasing physiologically active components, thereby completed the present invention. Accordingly, the present invention relates to a method for releasing a physiologically active component characterized by treating a disaccharide glycoside β-primeveroside and/or its analog, which are physiologically active component precursors, with β-xylosidase and/or β-glucosidase and thus releasing the physiologically active component.

### DETAILED DESCRIPTION OF THE INVENTION

Unless otherwise noted, enzyme activities are expressed herein in the data determined by the following methods.

### (1) β-xylosidase activity

The activity was measured by using an automatic chemical analyzer (Model TBA-20R manufactured by Toshiba).

45 µl of an enzyme sample was mixed with 200 µl of a solution of p-nitrophenyl (pNP) xyloside (manufactured by Sigma), employed as the substrate, dissolved in 2 mM acetate buffer (pH 5.5) and the resultant mixture was reacted at 37°C for a cycle time of 30.0 sec. After adding 250 µl of sodium carbonate thereto, the absorbance was measured at 412 nm. As the blank measurement originating in the sample, the procedure was repeated but using the 20 mM acetate buffer (pH 5.5) as a substitute for the substrate solution.

An amount of the enzyme which increases the absorbance of 1 under these conditions was defined to be one unit (1 AU).

### (2) β-Glucosidase activity

The activity was measured by using an automatic chemical analyzer (Model TBA-20R manufactured by Toshiba).

45 µl of an enzyme sample was mixed with 200 µl of a solution of p-nitrophenyl (pNP) glucoside (manufactured by Merck), employed as the substrate, dissolved in 2 mM acetate buffer (pH 5.5) and the resultant mixture was reacted at 37°C for a cycle time of 30.0 sec. After adding 250 µl of sodium carbonate thereto, the absorbance was measured at 412 nm. As the blank measurement originating in the sample, the procedure was repeated but using the 20 mM acetate buffer (pH 5.5) as a substitute for the substrate solution.

As amount of enzyme which increases the absorbance of 1 under these conditions was defined to be one unit (1 AU).

Next, the present invention will be described in greater detail. The present inventors made examinations on enzymes capable of cleaving one or more monosaccharide unit from β-primeveroside or disaccharide glycosides analogous to β-primeveroside and thus releasing aglycons, and commercially available enzyme preparations capable of releasing aglycons when employed in combination. Further, they attempted to screen microorganisms capable of producing these enzyme preparations. The glycoside to be treated in the present invention includes not only β-primeveroside and disaccharide glycosides analogous to β-primeveroside as described above but those wherein more than two saccharides are bonded. The term "disaccharide glycosides analogous to β-primeveroside" as used herein means disaccharide glycosides having glucose in the aglycon side such as apiofuranosyl-β-D-glucopyranoside and arabinofuranosyl-β-D-glucopyranoside.

An enzyme having the properties needed in the present invention and a microorganism capable of producing an enzyme usable in the invention can be screened as follows. In case of a microbial strain, it as cultured in an appropriate liquid medium. In case of an enzyme preparation, it is suspended and/or diluted in an appropriate buffer. Then, the microorganism can be screened by using, for example, pNP-primeveroside as a substrate with the use as an indication of the amount of the pNP released.

The pNP-primeveroside to be used as the substrate can be synthesized by, for example, reacting pNP-glucoside (manufactured by Merck) with xylooligosacchairde (manufactured by Wako Pure Chemical Industries) by using an enzyme, xylosidase (manufactured by Sigma), and thus transferring a xylose residue to the pNP-glucoside via a β-1,6 bond.

By screening commercially available enzyme preparations, the inventors found out that Pectinase G originating in *Aspergillus pulverulentus* (manufactured by Amano Pharmaceutical Co., Ltd.) contained enzymes capable of cleaving one or more monosaccharide unit from β-primeveroside or its analog to thereby release a physiologically active component.

The present inventors further confirmed that β-xylosidase and/or β-glucosidase participated in this function. That is, they have confirmed a functional mechanism wherein xylose is released from β-primeveroside. which is a disaccharide glycoside, under the action of β-xylosidase and subsequently glucose is released from the monosaccharide glycoside under the action of β-glucosidase, thereby releasing a physiologically active component.

In the present invention, it is possible to use a mixture of an enzyme preparation having a β-xylosidase activity with another enzyme preparation having a β-glucosidase activity originating respectively in different sources.

The β-xylosidase and β-glucosidase that constitute the enzyme composition to be used in the present invention may originate in arbitrary sources including animals, plants and microorganisms without restriction. For example, β-xylosidases originating in microorganisms of the genus *Penicillium* and pond snail (*Charonia lampas*) and β-glucosidases originating in apricot seed, cycad seed and *Aspergillus niger* may be used. Needless to say, it is also possible to use those separated and purified from commercially available enzyme preparations, for example, those used in the Examples shown hereinafter.

With respect to the mixing ratio, the ratio of β-xylosidase activity : β-glucosidase activity ranges from 10:0 to 0:10, preferably form 8:2 to 2:8 and still preferably form 6:4 to 4:6. It is also possible that these enzyme compositions are reacted successively so as to release the aimed physiologically active component form the glycoside. The present inventors have further found out that β-xylosidase and β-glucosidase can each act alone on β-primeveroside or its analog to cleave one or more monosaccharide unit thereby releasing the physiologically active component.

It is also possible to newly obtain the enzyme composition to be used in the invention from natural substances by using the screening method as described above. Namely, an enzyme composition usable in the present invention can be obtained by incubating and extracting the obtained microorganism in a conventional manner. Alternatively, an enzyme composition usable in the invention can be produced by various gene manipulation techniques.

That is, the production method usable in the present invention involves those with the use of mutants of microorganisms capable of producing β-xylosidase and/or β-glucosidase, various microorganisms or cells (for example, yeast cells, bacterial cells, higher plant cells and animal cells) having been modified by the DNA recombination method so as to produce β-xylosidase and/or β-glucosidase. In case where the β-xylosidase and/or β-glucosidase-productivity can be imparted by transferring β-xylosidase and/or β-glucosidase genes, the microorganism serving as the host may have no β-xylosidase and/or β-glucosidase productivity.

To produce β-xylosidase and/or β-glucosidase by using, for example, a microorganism, the microorganism may be incubated by an appropriate method under appropriate conditions without restriction. To incubate such a microorganism, either the liquid culture method or the solid culture method may be used, though the liquid culture method is preferred. The liquid culture may be carried out, for example, as follows.

Any medium may be used therefor, so long as the microorganism producing β-xylosidase and/or β-glucosidase can grow therein. For example, usable mediums include a medium containing carbon sources such as glucose, sucrose, soluble starch, glycerol, dextrin, molasses and organic acids, nitrogen sources such as ammonium sulfate, ammonium carbonate, ammonium phosphate, ammonium acetate, peptone, yeast extract, corn steep liquor, casein hydrolyzate, wheat bran and meat extract, and inorganic salts such as potassium salts, magnesium salts, sodium salts, phosphates, manganese salts, iron salts and zinc salts. It is also possible to add various inducers to the medium so as to produce and accumulate β-xylosidase and/or β-glucosidase therein.

The pH value of the medium may be regulated to about 3 to 8, preferably about 5 to 6. The incubation is carried out under aerobic conditions usually at about 10 to 50°C, preferably at about 30°C for 1 to 15 days, preferably for about 4 to 7 days. As the culture method, shaking culture method and the aerobic submerged culture method using a jar fermenter can be used. As a matter of course, these culture conditions may be appropriately varied depending on the microorganism or cells to be incubated. Namely, the culture conditions are not restricted, so long as the β-xylosidase and/or β-glucosidase usable in the present invention can be produced thereby.

From the culture medium thus obtained, the β-xylosidase or β-glucosidase may be isolated and purified by combining conventional procedures such as centrifugation, UF concentration, salting out and various chromatographic techniques (for example, ion exchange resin chromatography) to give purified β-xylosidase or β-glucosidase.

The obtained culture medium as it is may be used as the enzyme composition of the invention. Needless to say, the extent of purification of the culture medium may be appropriately varied depending on the purpose.

Next, various uses of the enzyme compositions of the invention will be described. The enzyme compositions usable in the present invention can be employed in, for example, enriching the scent, color and physiologically active components of vegetable materials and controlling the extraction efficiency of these components. Accordingly, the enzyme compositions are applicable to the production of, for example, foods, drinks, spices and perfumes with enriched scent. When adequately used in the process of manufacturing these products, moreover, the enzyme compositions make it possible to release undesirable smell components at an early state. Regarding colors, the enzyme compositions are usable in improving or developing the color of vegetable materials, foods and drinks and manufacturing pigments.

Also, the enzyme compositions can be used in decomposing and eliminating pigment precursors which are unfavorable from the viewpoint of quality control, as is similar to the case of scent components. With respect to physiologically active components, the enzyme compositions are usable in potentiating pharmacological components and useful physiologically active components of herbal medicine, herbs and other vegetable materials as well as in decomposing and eliminating unfavorable components.

Namely, these effects can be achieved by treating disaccharide glycosides such as primeverosides and analogs thereof with β-xylosidase and/or β-glucosidase.

Materials containing primeverosides or analogs thereof to be treated in the present invention may be arbitrary ones, so long as they can be treated with the enzyme compositions containing β-xylosidase and/or β-glucosidase used in the present invention. Examples thereof include foods, cosmetics, drugs, quasi drugs, agricultural chemicals and feeds. More particularly speaking, the invention is applicable to the production of foods, toiletry goods, and industrial products made of vegetable materials such as woodworks and tatami mats having various scents.

The subject to which the method of the present invention is desirably applied include scented foods. More particularly speaking, it may be used in the step of "withering" in the process of producing oolong tea or jasmine tea. Also, it is usable in enriching the scent of black tea leaves (for example, those for teabags produced by the CTC method) and enriching the scent of wine. Moreover, it is usable in sustaining the scent of cosmetics or sustaining the scent of perfumes, improving the smell of drugs and improving the pharmacological effects thereof.

Furthermore, it is usable in producing pigments. When the method of the present invention is employed in extracting alizarin (a dye) from ruberythric acid of *Rubia tinctorum*, the pigment can be more efficiently extracted.

In the utilization of the enzyme compositions usable in the present invention, the addition manner, the addition level and the reaction method may be appropriately varied depending on the form of the subjects to which the enzyme compositions are added.

As the concrete application method, the enzyme compositions are added to plant extracts or fermentation products containing scent component precursors and incubated. The culture conditions are not particularly restricted, so long as the enzyme compositions can act on the scent, pigment or physiologically active component precursors and release the same. Such conditions can be designed by those skilled in the art without undue labor. The concentrations of the aimed components can be elevated under these conditions.

It is also possible to use the enzymes of the present invention in elevating the concentrations of scent, pigment or physiologically active component precursors contained in plants. Since a plant contains precursors of these components, the concentrations, the contents of the scent, pigment or physiologically active components can be increased by adding an effective amount of the enzyme composition to the plant and growing the plant under such conditions allowing the hydrolysis of the precursors. Also, use of the enzyme compositions of the invention makes it possible to regulate the time of the formation of scent, pigment or physiologically active components in a target plant.

The reaction conditions for the method of the present invention are not particularly limited, as long as the reaction to release one or more monosaccharide unit from a glycoside by the action of an enzyme composition can proceed. The reaction may be carried out in an aqueous solution, in an organic solvent (e.g., methyl acetate, ethanol, methanol), in a mixed solvent thereof, the solid-liquid ununiform system (substrate or enzyme is solid and medium is liquid), or the like, preferably in an aqueous solution. The concentration of the glycoside may be preferably about 1M or less, more preferably 0.1 M or less, and most preferably 4 mM or less. Each enzyme may be used preferably in an amount of 1% by weight or more, more preferably 10% by weight or more based on the amount of glycoside (preferably 0.01 to 0.1 units/mg substrate, more preferably 0.1 units to 1 units/mg substrate). The reaction may be carried out preferably at a temperature of from 0 to 65°C, more preferably from 20 to 65°C, most preferably from 30 to 50°C in a period of preferably from 1 hour to 3 days.

The present invention will be described in greater detail by reference to the following Examples, but it should be understood that the invention is not construed as being limited thereto.

### EXAMPLE 1

3.6 kg of Pectinase G™ (manufactured by Amano Pharmaceutical Co., Ltd., originating in *Aspergillus pulverrulentus*) was dissolved in 20 mM phosphate buffer (pH 6.0) to give 17.5 L of a solution. After adding 7.0 kg of ammonium sulfate (65% saturation), the mixture was stirred and centrifuged and 17.8 L of the supernatant was collected. To this supernatant, 3.65 kg of ammonium sulfate (95% saturation) was further added and the obtained mixture was stirred and centrifuged. 16.7 L of the supernatant was collected and concentrated to 210 ml with an ultrafiltration membrane (molecular weight cut: 6,000) followed by freeze-drying. Then the β-xylosidase activity and β-glucosidase activity of this freeze-dried product were measured.

As a result, it showed a β-xylosidase activity of 7.12 units/mg and a β-glucosidase activity of 0.12 units/mg.

A 50 mg portion of the freeze-dried product obtained above was dissolved in 10 ml of 25 mM bis-Tris buffer (pH 7.1), developed on an anion exchange Mono-P column (5 x 200 mm) having been equilibrated with 25 mM bis-Tris buffer (pH 7.1) and eluted with Polybuffer (manufactured by Pharmacia) to give a partly purified enzyme preparation of β-xylosidase. The β-xylosidase activity was 82.2 units/mg while the β-glucosidase activity was 1.01 units/mg.

### EXAMPLE 2

1.2 g of Pectinase G™ (manufactured by Amano Pharmaceutical Co., Ltd., originating in Aspergillus pulverrulentus) was dissolved in 12 ml of 20 mM phosphate buffer (pH 6.0). After centrifuging, 28 ml of 10% saturation ammonium sulfate/20 mM phosphate buffer (pH 6.0) was added (70% saturation) to 12 ml of the supernatant. After stirring, the mixture was centrifuged and the precipitate thus formed was recovered. This precipitate was dissolved in 12 ml of 35% saturation ammonium sulfate/20 mM phosphate buffer (pH 6.0). After centrifuging, the supernatant was collected. Then, the β-glucosidase activity and β-xylosidase activity of this supernatant were measured.

As a result, it showed a β-glucosidase activity of 66.6 units/mg and a β-xylosidase activity of 2.42 units/mg.

A 10 ml portion of the supernatant obtained above was developed on a Phenyl Sepharose column (16 x 100 mm) having been equilibrated with 35% saturation ammonium sulfate/20 mM phosphate buffer (pH 6.0) and eluted with 20 mM phosphate buffer (60%) to give a partly purified enzyme preparation of β-glucosidase. The β-glucosidase activity was 24.1 units/mg while the β-xylosidase activity was 0.18 units/mg.

### EXAMPLE 3

The partly purified β-xylosidase obtained in Example 1 was diluted with 20 mM acetate buffer (pH 5.5) so as to give a β-xylosidase activity of 5.0 units/ml. Further, the partially purified β-glucosidase obtained in Example 2 was diluted with 20 mM acetate buffer (pH 5.5) to give a β-glucosidase activity of 5.0 units/ml. By using these enzyme solutions and pNP-primeveroside, a reaction with β-xylosidase alone, a reaction with β-glucosidase alone and another reaction wherein β-glucosidase was added after the completion of the reaction with β-xylosidase alone were performed in the following manner. Then, the amounts of pNP thus released were compared.

The reaction with β-xylosidase alone was carried out in the following manner. 0.2 ml of a 10 mM pNP-primeveroside solution in 20 mM acetate buffer (pH 5.5) was mixed with 0.2 ml of the partly purified β-xylosidase. After 5 minutes, a 0.05 ml portion of the reaction mixture was sampled and mixed with 0.025 ml of 20 mM acetate buffer (pH 5.5) and 0.075 ml of 0.5 M sodium carbonate. Then the absorbance of the obtained mixture was measured at 420 nm (reaction for 5 minutes). Simultaneously (i.e., after 5 minutes), a 0.15 ml portion of the reaction mixture was sampled and mixed with 0.075 ml of 20 mM acetate buffer (pH 5.5) and the reaction was continued. 5 and 15 minutes thereafter, 0.1 ml portions of the reaction mixture were each sampled and mixed with 0.1 ml of 0.5 M sodium carbonate followed by the measurement of the absorbance at 420 nm (reactions for 10 and 20 minutes).

The reaction with β-glucosidase alone was carried out in the following manner. 0.15 ml of a 10 mM pNP-primeveroside solution in 20 mM acetate buffer (pH 5.5) was mixed with 0.15 ml of the partly purified β-glucosidase. After 5 minutes, a 0.05 ml portion of the reaction mixture was sampled and mixed with 0.025 ml of 20 mM acetate buffer (pH 5.5) and 0.075 ml of 0.5 M sodium carbonate. Then the absorbance of the obtained mixture was measured at 420 nm (reaction for 5 minutes). Simultaneously (i.e., after 5 minutes), a 0.2 ml portion of the reaction mixture was sampled and mixed with 0.1 ml of 20 mM acetate buffer (pH 5.5) and the reaction was continued. 5 and 15 minutes thereafter, 0.1 ml portions of the reaction mixture were each sampled and mixed with 0.1 ml of 0.5 M sodium carbonate followed by the measurement of the absorbance at 420 nm (reactions for 10 and 20 minutes).

The reaction wherein β-glucosidase was added after the completion of the reaction with β-xylosidase alone was carried out in the following manner. 5 minutes after the reaction with β-xylosidase alone, a 0.15 ml portion of the reaction mixture was sampled and 0.075 ml of the partially purified β-xylosidase was added thereto and the reaction was continued. 5 and 15 minutes thereafter, 0.1 ml portions of the reaction mixture were each sampled and mixed with 0.1 ml of 0.5 M sodium carbonate followed by the measurement of the absorbance at 420 nm (reactions for 10 and 20 minutes).

In each case, the procedure was repeated but using a pre-heated (100°C, 10 minutes) sample to give a blank value. Table 1 shows the results.

**TABLE 1**

| | ΔOD420 | | |
|---|---|---|---|
| Time (minutes) | β-Glc 5AU/ml | β-Xyl 5AU/ml | β-Xyl5AU/ml + β-Glc5AU/ml (after 5 minutes) |
| 0 | 0.000 | 0.000 | 0.000 |
| 5 | 0.022 | 0.005 | 0.005 |
| 10 | 0.037 | 0.013 | 0.272 |
| 20 | 0.061 | 0.043 | 0.874 |

As Table 1 clearly shows, pNP was released in an increased amount by the reaction wherein β-glucosidase was added after the completion of the reaction with β-xylosidase alone, compared with the reactions with the use of β-xylosidase or β-glucosidase alone. Based on these results, it was confirmed that pNP-primeveroside is decomposed into pNP-glucoside and xylose by β-xylosidase and the pNP-glucoside, which is the substrate of β-glucosidase, is then decomposed into pNP and glucose by β-glucosidase.

### EXAMPLE 4

The partly purified β-xylosidase obtained in Example 1 was diluted with 20 mM acetate buffer (pH 5.5) to give an activity of 5.0 units/ml. Further, the partially purified β-glucosidase obtained in Example 2 was diluted with 20 mM acetate buffer (pH 5.5) to give an activity of 5.0 units/ml. By using these enzyme solutions, samples having β-xylosidase activity:β-glucosidase activity ratios of 10:0, 9:1, 8:2, 7:3, 6:4, 5:5, 4:6, 3:7, 2:8, 1:9 and 0:10 were prepared.

0.05 ml of each sample was mixed with 0.05 ml of a 10 mM pNP-primeveroside solution in 20 mM acetate buffer (pH 5.5) and incubated at 37°C for 20 minutes. After adding 0.1 ml of 0.5 M sodium carbonate, the absorbance was measured at 420 nm. The procedure was repeated but using a pre-heated (100°C, 10 minutes) sample to give a blank value. Table 2 shows the results.

**TABLE 2**

| Ratio of activity β-Xyl:β-Glc | Sample OD420 | Blank OD420 | ΔOD420 | Relative ratio |
|---|---|---|---|---|
| 10:0 | 0.116 | 0.022 | 0.094 | 16.7 |
| 9:1 | 0.318 | 0.015 | 0.303 | 53.8 |
| 8:2 | 0.457 | 0.015 | 0.442 | 78.5 |
| 7:3 | 0.544 | 0.023 | 0.521 | 92.5 |
| 6:4 | 0.570 | 0.023 | 0.547 | 97.2 |
| 5:5 | 0.580 | 0.017 | 0.563 | 100 |
| 4:6 | 0.550 | 0.022 | 0.528 | 93.8 |
| 3:7 | 0.498 | 0.028 | 0.470 | 83.5 |
| 2:8 | 0.415 | 0.032 | 0.383 | 68.0 |
| 1:9 | 0.299 | 0.035 | 0.264 | 46.9 |
| 0:10 | 0.136 | 0.044 | 0.092 | 16.3 |

As Table 2 clearly shows, pNP was released in the largest amount by using the sample with a β-xylosidase activity:β-glucosidase activity ratio of 5:5, i.e., 1:1.

### EXAMPLE 5

### Method for preparing eugenyl primeveroside (scent component precursor):

About 2 kg of fresh leaves of a sasanqua (*Camellia saccharidenqua*) were extracted with hot water at 100°C for 10 minutes and the extract was treated with a column packed with Diaion HP20 (manufactured by Mitsubishi Chemical Corporation) to thereby adsorb eugenyl primeveroside. Then the column was washed with deionized water and 20% methanol about twice as much as the bed volume. Next, the adsorbed eugenyl primeveroside was recovered with 100% methanol. The methanol solution containing the thus recovered eugenyl primeveroside was concentrated and the eugenyl primeveroside was crystallized and taken up with a glass filter.

### EXAMPLE 6

The partly purified β-xylosidase and the partly purified β-glucosidase, obtained respectively in Examples 1 and 2, were diluted with 20 mM acetate buffer (pH 5.5) to give a sample having a β-xylosidase activity:β-glucosidase activity ratio of 1:1, namely showing a β-xylosidase activity of 10.0 units/ml and a β-glucosidase activity of 10.0 units/ml.

0.5 ml of this enzyme solution was mixed with 0.5 ml of a 10 mM eugenyl primeveroside solution in 20 mM acetate buffer (pH 5.5) and incubated at 37°C. After 6 and 24 hours, samples were taken. After heating (100°C, 10 minutes) each sample to stop the reaction, pentanol was added thereto as an internal standard and the mixture was analyzed by gas chromatography (manufactured by Shimadzu Co.) As a result, each of the samples (6 and 24 hours) showed a peak within the eugenol detection time. It was also confirmed that the amount of the released eugenol increased with the passage of time.

When the enzyme solution was pre-heated. (100°C, 10 minutes) and then treated in the same manner, no eugenol was detected after 6 and 24 hours.

In a sensory test by using 10 panels, the characteristic scent of eugenol was noticeable in both samples (6 and 24 hours). Also, it was confirmed that the scent of eugenol was enhanced with the passage of time. When the enzyme solution was pre-heated (100°C, 10 minutes) and then treated in the same manner, no scent of eugenol was noticeable.

Based on these results, it has been confirmed that the composition containing β-xylosidase and β-glucosidase acts on the primeveroside glycoside, which is a natural substrate, to release the aglycon.

### EXAMPLE 8

1 ml of the enzyme solution obtained in Example 6 was mixed with 1 ml of a commercially available grape juice (fruit juice 100%, concentrated and reconstituted) and incubated at 37°C overnight (14 hours). When the scent was examined, this mixture showed a remarkably enhanced scent compared with one containing an acetate buffer in lieu of the enzyme preparation. When the enzyme solution was pre-heated (100°C, 10 minutes), no such effect was achieved.

### EXAMPLE 9

1 ml of the enzyme solution obtained in Example 6 was mixed with 1 ml of a commercially available orange juice (concentrated and reconstituted) and incubated at 37°C for 24 hours. Then, the scent formation was examined by a sensory test. As a result, it showed an enhanced scent of orange juice. When the enzyme solution was pre-heated (100°C, 10 minutes), no such effect was achieved.

According to the invention, it has been clarified that an enzyme composition containing β-xylosidase and/or β-glucosidase cleaves one or more monosaccharide unit from β-primeveroside or disaccharide glycosides analogous thereto to thereby release the aglycon. The enzyme compositions usable in the present invention, which can be supplied with the use of microbial sources, are widely applicable to, for example, various foods, drugs and quasi drugs. When used in foods, for example, these compositions can enhance or weaken the scent, pigment and physiologically active components.

While the invention has been described in detail and with reference to specific embodiments thereof, it will be apparent to one skilled in the art that various changes and modifications can be made therein without departing from the spirit and scope thereof.

This application is based on a Japanese patent application No. Hei.-11-075575, filed on March 19, 1999, incorporated herein by reference.

## Claims

1. A method of releasing one or more monosaccharide unit from a glycoside, which comprises treating said glycoside with an enzyme composition.

2. The method as claimed in claim 1, wherein said glycoside is a disaccharide glycoside.

3. The method as claimed in claim 2, wherein said disaccharide glycoside is at least one of β-primeveroside and a disaccharide glycoside which is analogous to β-primeveroside.

4. The method as claimed in any one of claims 1 to 3, wherein said enzyme composition is obtainable from a microorganism or a plant.

5. The method as claimed in any one of claims 1 to 3, wherein said enzyme composition comprises at least one of β-xylosidase and β-glucosidase.

6. The method as claimed in any one of claims 1 to 3, wherein said disaccharide glycoside is a scent component precursor, a pigment component precursor or a physiologically active component precursor.

7. The method as claimed in claim 4, wherein said disaccharide glycoside is a scent component precursor, a pigment component precursor or a physiologically active component precursor.

8. The method as claimed in claim 5, wherein said disaccharide glycoside is a scent component precursor, a pigment component precursor or a physiologically active component precursor.

9. The method as claimed in claim 6, wherein said scent component precursor, pigment component precursor or physiologically active component precursor originates in a plant.

10. The method as claimed in claim 7, wherein said scent component precursor, pigment component precursor or physiologically active component precursor originates in a plant.

11. The method as claimed in claim 8, wherein said scent component precursor, pigment component precursor or physiologically active component precursor originates in a plant.
